# EUROPEAN PATENT APPLICATION

(11) **EP 1 787 674 A1**
(43) Date of publication of application: **23.05.2007**
(21) Application number: 05767269.3
(22) Date of filing: 27.07.2005
(51) Int. Cl.: A61M 25/00

(54) **CATHETER TUBE FOR MEDICAL TREATMENT AND METHOD OF MANUFACTURING THE SAME**

(30) Priority: 10.08.2004 JP 2004233172; 10.08.2004 JP 2004233336
(71) Applicant: Kaneka Corporation, Kita-ku Osaka-shi, Osaka 530-8288 (JP)
(72) Inventor: MURATA, Takahiro, c/o KANEKA CORPORATION, Settsu-shi, Osaka 566-0072 (JP); MIHAYASHI, Tsuyoshi, c/o KANEKA CORPORATION, Settsu-shi, Osaka 566-0072 (JP)
(74) Representative: Gille Hrabal Struck Neidlein Prop Roos
(86) International application number: PCT/JP2005/013761
(87) International publication number: WO 2006/016481

(57) **Abstract**

A catheter tube for medical treatment having a high degree of freedom in having its rigidity and flexibility changed stepwise and enabling the setting of a bending rigidity changing position according to a variety of access routes and a method of manufacturing the catheter tube. The catheter tube comprises an inner layer tube, a reinforcement material layer, a marker, and an outer layer tube which are formed integrally with each other. The catheter tube is so formed that a line body forming the reinforcement material layer is densely wound at its tip part, the marker formed of an X-ray impermeable metal wire adjacent to the tip thereof is also densely wound, and comprises a leading-edge part where the reinforcement material layer and the marker are not present. Accordingly, the bending rigidity of the catheter tube from the base part to ward the tip part is stepwise or continuously decreased by the presence of the reinforcement material layer and the outer layer tube. The winding of the line body is terminated at the base part after its interval and tilt angle are changed at positions between the densely wound portion of the tip part and a portion near the base part, and the outer layer tube has a stepwise-changing hardness arrangement. As a result, the catheter tube can be set to a variety of bending rigidity changing positions.

## Description

### TECHNICAL FIELD

The present invention relates to a catheter tube for medical treatment superior in positioning efficiency, torque-transmitting efficiency, and kink resistance that has a higher latitude in adjusting the tilt of rigidity and flexibility and the adjusting the rigidity/flexibility balance according to access route, and a method of producing the same. The present invention also relates to a catheter tube for medical treatment superior in X-ray visibility and flexibility in the distal region and a method of producing the same.

### BACKGROUND ART

Catheter tubes are hollow medical devices that are used as inserted in the cavity, duct, blood vessel, and others in the body, specifically for selective injection of angiographic agent or others, removal of blood clot, recirculation of clogged vessel, vasodilation, and the like , and are normally tube-shaped. Such a catheter demands superior usability allowing rapid, accurate, and selective insertion thereof, for example, into thin complicated-patterned blood vessel.

More specifically as for operability, such a catheter tube demands favorable positioning efficiency allowing transmission of the surgeon's operation, such as insertion and withdrawal thereof into or out of the blood vessel, from the proximal region to the distal region and favorable pressure resistance, for example, when a drug solution flows inside. It also demand favorable torque-transmitting efficiency allowing reliable transmission of the torque applied in the proximal region of catheter tube and favorable insertion efficiency allowing transmission of the surgeon's force pushing the catheter tube into the blood vessel from the proximal end to the distal end. It also demand favorable guide wire compatibility, i.e., lubricity of the inner surface of the catheter, allowing insertion and withdrawal of the catheter tube along the guide wire inserted into the blood vessel in the complicated shape smoothly without damaging the blood vessel inner wall or the like, and favorable affinity to blood and organs on the outer surface of the catheter. In addition, it also demands favorable kink resistance prohibiting folding of the catheter tube in the curved or bent area of blood-vessel when the distal end of catheter tube reaches a desired position and then the guide wire is removed, and favorable distal-region flexibility keeping the shape favorable for the blood vessel.

It is generally known that a structure having a relatively rigid proximal region and a distal region gradually becoming more flexible is favorable for giving properties satisfying the requirements above.

To obtain a catheter tube having such properties described above, known is a method of forming a catheter tube by winding an inner layer tube with a strand in the coil shape as a reinforcement material layer or covering the inner layer with an outer layer of braid.

As the catheter tube in which a strand is wound around the inner layer tube in the coil shape as the reinforcing material layer, Patent Document 1 discloses a catheter tube having a catheter main body comprising flexible inner and outer tubes are partially connected to each other via a reinforcing material layer, wherein: the reinforcement material layer is made of a strand woven in the lattice-shaped; and a region higher in flexural rigidity and a region smaller in flexural rigidity are formed by changing the tilt angle of the strand to the axis of the catheter main body continuously or stepwise in the axial direction of the catheter main body or the interval between the lattice points of the strand continuously or stepwise in the axial direction of the catheter main body.

However, it is possible to form a high-rigidity proximal region and a high-flexibility distal region in the catheter tube above, but the latitude in controlling the tilt in its rigidity and flexibility is smaller, and it is necessary to make an catheter tube having relatively thicker inner and outer diameters to attain favorable flexibility tilt, and decrease in the thickness of catheter tube wall results in deterioration in kink resistance. In addition, the rigidity/flexibility balance of the catheter tube is not intended to be adjustable according to access route. In addition, the catheter tube is lower in flexibility in the catheter distal region.

Also disclosed as the catheter tube having an inner layer tube wound with a strand in a coil shape as the reinforcement material layer in Patent Document 2 is a catheter tube having a tube-shaped part consisting of a proximal end, a distal end, and a hollow passage extending between these ends, wherein: the long tube-shaped part has an outer tube-shaped cover of a first cover material; an inner tube-shaped liner of a first liner material coaxial therewith; and at least one first ribbon-reinforcing material wound around the inner tube-shaped liner spirally or coaxially and covered with the outer tube-shaped cover.

However, even in the configuration, the latitude in controlling the tilt in its rigidity and flexibility is lower, and the productivity is also lower, because the cutting edge often caused a problem of breakage of the inner tube-shaped liner and the outer tube-shaped cover by the elasticity force of the ribbon-reinforcing material during production. In addition, the rigidity/flexibility balance of the catheter tube is not intended to be adjustable according to access route. Further, the catheter tube is lower in flexibility in the catheter distal region.

Alternatively, Patent Document 3 discloses a flexible tube having a reinforcement material layer inside, in which almost cylindrical flexible inner and outer tubes are connected to each other via the reinforcement material layer with the inner tube inside the outer tube, wherein: the reinforcement material layer is a knitted braid of one or more flat square strands having a tensile strength of 500 to 2,000 MPa; and the angle of each flat rectangular strand to the axis direction of the inner tube changes stepwise or continuously almost along the axis line.

However, although it is possible to form a high-rigidity proximal region and a high-flexibility distal region in the catheter tube, the latitude in controlling the tilt in rigidity and flexibility is lower and it is needed to make catheter tube relatively larger in outer diameter for exhibition of the tilt in flexibility, and thus, the catheter tube has lower kink resistance when the thickness of the catheter tube wall is reduced. In addition, the rigidity/flexibility balance of the catheter tube is not intended to be adjustable according to access route. Further, the catheter tube is lower in flexibility in the catheter distal region.

Yet alternatively, Patent Document 4 discloses a catheter comprising a flexible tube-shaped catheter main body and a reinforcing coil embedded in the wall of the catheter main body, wherein: the catheter main body further has a first region at the distal end of the catheter and a second region at a position closer to the proximal end from the first region; the coil extends from the first region to the second region; the coil is wound at a relatively large winding pitch over the entire length in the second region; the coil is wound at a relatively smaller winding pitch between neighboring coils over the entire length in the first region; the coil winding pitch declines gradually in the direction toward the distal end; and the rigidity of the catheter is smaller in the first region than in the second region.

However, although it is possible to form a high-rigidity proximal region and a high-flexibility distal region in the catheter tube and keep a favorable balance in flexural rigidity, the rigidity/flexibility balance of the catheter tube is not intended to be adjustable according to access route. Further in the catheter tube, the entire reinforcing coil is a radiopaque metal wire; the flexibility of the distal region is insufficient; and the X-ray visibility is excessively high, causing troubles in decision making by surgeons during operation.

Yet alternatively, Patent Document 5 discloses a guide catheter comprising a base region, a distal region, a distal tip in the distal region having a first lumen between the base region and the distal region, wherein: the guide catheter further has an inner tube-shaped layer having a base region, a distal region, and a second lumen extending between them, a radiopaque marker placed on the inner tube-shaped layer close to the distal region of the inner tube-shaped layer, and a reinforcing layer having a base region and a distal region; the second lumen communicates with the first lumen; the distal region of the inner tube-shaped layer extends to the base region of the distal tip; the reinforcing layer is formed on the inner tube-shaped layer; and the distal region of the reinforcing layer terminates at the position close to the radiopaque marker.

Although a wire is wound or braided around the inner layer tube and an additional outer layer is placed thereon in forming the reinforcing layer in the catheter tube, the properties of the high-rigidity proximal region and the high-flexibility distal region are shown only insufficiently and, in addition, the rigidity/flexibility balance of the catheter tube is not intended to be adjustable according to access route. Further, its radiopaque marker seemingly formed with a resin tube containing a blended metal powder is placed between the tip region and the main body region, but the radiopaque marker and the tip region may be separated from each other when the adhesiveness is insufficient then.

Alternatively for preparing a catheter in which a braid of strand is wound around an inner layer tube as the reinforcing material layer, Patent Document 6 discloses a method of producing a catheter comprising: forming a torque-transmitting region by covering the outer surface of a thermoplastic tube containing an inserted metal core wire with a metal braid; forming multiple insertion distal regions having a constant width at a particular interval in the tube machine direction by removing part of the braid intermittently in the machine direction by irradiation of a laser beam at a wavelength of 1.06 µm from outside; withdrawing the metal core wire; and forming the insertion distal regions continuously in the distal regions of torque transmission regions by dividing the tube into pieces at the terminal of each insertion distal region.

However, the step of removing part of the braid intermittently in the machine direction by irradiation of a laser beam at a wavelength of 1.06 µm is very complicated. The method is also lower in productivity, because, when a torque transmission region is formed continuously by applying a metal braid continuously over the outer surface of a thermoplastic tube having an inserted metal core wire and embedding the braid into the outer surface of the tube by softening by heating to a depth of about 1/2 to 1/5 of the thickness in the downstream step, the metal braid may cause problems such as ejection of the metal braid out of the tube surface caused by the bending of cut edge due to its elastic force during embedment of the braid by heat softening of the tube. It is also difficult to control the tilt in rigidity and flexibility sufficiently. In addition, the rigidity/flexibility balance of the catheter tube is not intended to be adjustable according to access route.
Patent Document 1: Japanese Patent No. 3310031
Patent Document 2: Japanese Patent No. 2672714
Patent Document 3: Japanese Unexamined Patent Publication No. 2001-299922
Patent Document 4: Japanese Unexamined Patent Publication No. 2001-218851
Patent Document 5: Japanese Unexamined Patent Publication No. 2002-532164
Patent Document 6: Japanese Unexamined Patent Publication No. 2000-225194

### SUMMARY OF THE INVENTION

### Problems to be Solved by the Invention

An object of the present invention is to provide a catheter tube for medical treatment superior in positioning efficiency, torque-transmitting efficiency, flexibility, kink resistance, pressure resistance, insertion efficiency, X-ray visibility, and others, and a method of producing the same.

In particular, because the medical catheter tube according to the present invention is used in various affected parts and the access routes thereto are also diversified, it provides a catheter tube for medical treatment that has a higher latitude in adjusting the tilt of rigidity and flexibility and adjusting the rigidity/flexibility balance according to access route, and a method of producing the same.

Further, the catheter tube according to the present invention contains a reinforcement material layer distal end and its neighboring marker made of a densely wound strand and is advantageous in its favorable X-ray visibility and flexibility in the distal region, and the present invention also discloses a method of producing the same.

It also discloses a method of producing a thin-outer diameter and large-inner diameter catheter tube, i.e., a thin catheter tube for medical treatment.

### Means to Solve the Problems

Accordingly, the present invention (1) relates to
a catheter tube for medical treatment, comprising an inner layer resin tube, a reinforcement material layer of a strand for adjustment of flexibility and improvement in kink resistance, torque-transmitting efficiency, and insertion efficiency wound around the inner layer tube, a marker of a radiopaque metal wire wound around the inner layer tube, and an outer layer resin tube covering the reinforcement material layer and the marker, wherein:
the catheter tube includes a distal area and a proximal area;
the distal area includes a tip region;
the proximal area includes a distal region, an intermediate region, and a proximal region;
in the proximal area,
the strand of reinforcement material layer is wound densely in the distal region, and the interval and tilt angle of winding are changed at least partially and the winding extends to the proximal region of the intermediate region;
in the distal area,
the marker is placed in the proximal region of the distal area, and the strand constituting the marker is wound densely, and
the strand is not wound in the region of the distal area other than the marker region; and
the flexural rigidity declines stepwise or continuously in the direction from the proximal region to the distal region in the proximal area because of presence of the reinforcing material layer.

The present invention (2) relates to
a catheter tube for medical treatment, comprising an inner layer resin tube, a reinforcement material layer of a strand for adjustment of flexibility and improvement in kink resistance , torque-transmitting efficiency, and insertion efficiency wound around the inner layer tube, a marker of a radiopaque metal wire wound around the inner layer tube, and an outer layer resin tube covering the reinforcement material layer and the marker, wherein:
the catheter tube includes a distal area and a proximal area;
the distal area includes a tip region;
the proximal area includes a distal region, an intermediate region, and a proximal region;
in the proximal area,
the strand constituting the reinforcement material layer is wound densely in the distal region,
and the winding extends to the proximal region while the interval and tilt angle of winding are changed at least partially in the intermediate region, and turns from the proximal region, then turns again from the distal end of the proximal region, and terminates in the proximal region;
in the distal area,
the marker is placed in the proximal region of the distal area, and the strand constituting the marker is wound densely, and
the strand is not wound in the region of the distal area other than the marker region; and
the flexural rigidity declines stepwise or continuously in the direction from the proximal region to the distal region in the proximal area because of presence of the reinforcing material layer.

The present invention (3) relates to
the catheter tube for medical treatment according to (1) or (2), wherein:
the catheter tube in the intermediate region of the proximal area includes a first intermediate area in the distal end side and a second intermediate area in the proximal region side;
the strand of reinforcement material layer is wound at a constant tilt angle and/or a constant interval in the first intermediate area; and
the strand is wound at an tilt angle and/or a interval changing continuously or stepwise in the second intermediate area.

The present invention (4) relates to
the catheter tube for medical treatment according to (1) or (2), wherein the Shore D hardness of the resin tubes of the outer layer tube declines continuously or stepwise in the direction from the proximal region to the distal region.

The present invention (5) relates to
the catheter tube for medical treatment according to (4), wherein the rigidity/flexibility balance of the catheter tube is adjusted arbitrarily by winding of the wire of reinforcement material layer and stepwise decrease in the Shore D hardness the resin tubes constituting the outer layer tube from the proximal region to the distal region.

The present invention (6) relates to
the catheter tube for medical treatment according to (1) or (2), wherein the inner- and outer-layer tubes are connected to each other via the reinforcement material layer and the marker.

The present invention (7) relates to
the catheter tube for medical treatment according to (1) or (2), wherein the inner layer tube is made of a resin lubricant to the guide wire or the like penetrating therein.

The present invention (8) relates to
the catheter tube for medical treatment according to (1) or (2), wherein the outer layer tube is molded into a rounded or tapered shape while the outer diameter thereof is changed and/or the inner and outer diameters of the inner layer tube changed in the tip region of the distal region.

The present invention (9) relates to
the catheter tube for medical treatment according to (1) or (2), wherein the outer layer tube is made hydrophilic by coating.

The present invention (10) relates to
a method of producing the catheter tube for medical treatment according to (1) or (2), comprising:
preparing an inner layer tube;
forming a marker on the inner layer tube;
winding a strand fed from a strand-supplying unit around the outer surface of the inner layer tube and forming a reinforcement material layer by changing the relative traveling speed and/or the relative traveling direction of the inner layer tube and the strand-supplying unit, and/or changing the relative traveling direction of the inner layer tube and the strand-supplying unit , while changing the tilt angle and interval of the strand to the catheter tube axis continuously and/or stepwise along the axial direction of the catheter tube; and
covering the inner layer tube, the marker, and the reinforcement material layer with an outer layer tube.

The present invention (11) relates to
the method of producing a catheter tube according to (10), wherein:
two or more resin tubes are used for the outer layer tube;
the resin tubes respectively have a Shore D hardness of two or more; and
the resin tubes are placed around the structure having a reinforcement material layer and a marker formed on the outer surface of inner layer tube, forming the outer layer tube in such a manner that the Shore D hardness of the resin tubes of the catheter tube decline stepwise in the direction from the proximal region to the distal region.

The present invention (12) relates to
the method of producing a catheter tube according to (11), further comprising:
preparing the outer layer tube by placing the resin tubes in order for preparation of the outer layer tube;
covering and heating all of the resin tubes with a shrink tube, and
integrating the inner layer tube, the reinforcing material layer, the marker and the outer layer tube;
then, molding the tip region of the outer layer tube into a rounded or tapered shape, and
obtaining a catheter tube for medical treatment by removing the shrink tube.

The present invention (13) relates to
the method of producing a catheter tube according to (10), wherein the outer layer tube is formed by coating one or more resins on the structure having a reinforcement material layer and a marker on the outer surface of the inner layer tube by extrusion in such a manner that the Shore D hardness becomes 2 or more and declines thereon in the direction from the proximal region to the distal region.

### Advantageous effects of the invention

Advantageously by the means to solve the problems described above, the present invention provides a catheter tube for medical treatment superior in positioning efficiency with favorable compatibility with the guide wire that has favorable torque-transmitting efficiency when the torque is applied by a surgeon, shows change in flexibility continuously in the direction from the proximal region to the distal region, and has higher latitude in adjusting rigidity and flexibility, favorable efficiency in adjusting the rigidity/flexibility balance according to access route, kink resistance prohibiting crimping when deformed in a complicated way, guide wire compatibility, and productivity. It also provides a catheter tube having favorable X-ray visibility and favorable flexibility in the distal region.

The hardness of the proximal region may increase when the strand of reinforcement material layer is wound with turning, but the invention also provides a catheter tube for medical treatment having a higher torque-transmitting efficiency.

### BRIEF DESCRIPTION OF THE DRAWINGS

Figure 1 shows an inner layer tube covering a metal core wire in the first and second aspects of the present invention.
Figure 2 shows the state when the winding initial end of the strand is fixed with a shrink tube and both ends of the core wire are set to a winder in the first and second aspects of the present invention.
Figure 3 shows the state when the strand is wound densely in a small region close to the initial end by the winder in the first and second aspects of the present invention.
Figure 4 shows the state when the strand is wound around the inner layer tube at a constant tilt angle or constant interval and the tilt angle or interval is changed continuously or stepwise in the region closer to the proximal region in the first and second aspects of the present invention.
Figure 5 shows the state when the end of the strand is covered with a shrink tube and fixed by heat shrinkage and then the strand is cut, in the first aspect of the present invention.
Figure 6 shows the state when four kinds of resin tubes different in Shore D hardness for the outer layer are placed densely in the first aspect of the present invention.
Figure 7 shows the state when the shrink tube at the winding initial end of the strand of reinforcement material layer is removed in the first aspect of the present invention.
Figure 8 shows the state when a radiopaque marker is placed in the first aspect of the present invention.
Figure 9 shows the state when the length of the inner layer tube distal region is adjusted in the first aspect of the present invention.
Figure 10 shows the state when the outer layer resin tube is slided to the position of the inner layer tube distal region or a position slightly closer to the proximal end in the first aspect of the present invention.
Figure 11 shows the state when the shrink tube is placed around the outer layer resin tube to the end in the first aspect of the present invention.
Figure 12 shows the state when the distal region of the outer layer resin tube is processed into a rounded shape with the position of the distal region almost identical with the position of the distal region of the inner layer tube in the first aspect of the present invention.
Figure 13 shows the distal end of the tube composite and a mold for heating the distal end in the first aspect of the present invention.
Figure 14 shows the state when the distal end of the tube composite is placed and molded under heat in the distal region-molding mold in the first aspect of the present invention.
Figure 15 shows a catheter tube obtained in the first aspect of the present invention.
Figure 16 shows the structure when the outer layer is removed in the distal region of Figure 15 in the first aspect of the present invention.
Figure 17 is a schematic diagram showing the rigidity/flexibility balance of the catheter tube in the first aspect of the present invention.
Figure 18 shows the state when multiple structures having an inner layer tube wound by a reinforcement material layer connected to each other in series by welding of the metal core wire that are wound around a reel in the first aspect of the present invention.
Figure 19 shows the state when the outer layer is formed by switching extrusion in the first aspect of the present invention.
Figure 20 shows the state when the shrink tube fixing the winding initial end of the strand of reinforcement material layer in the distal region is removed and the length of the distal region of the inner layer tube is adjusted in the first aspect of the present invention.
Figure 21 shows the state when a radiopaque marker is placed in the first aspect of the present invention.
Figure 22 shows the state when a resin tube for distal outer layer is placed and covered with a shrink tube in the first aspect of the present invention.
Figure 23 shows a catheter tube obtained in the first aspect of the present invention.
Figure 24 shows the state when the strand is wound with turning in the direction toward the distal end in the second aspect of the present invention.
Figure 25 shows the state when the strand is wound with turming in the direction toward the proximal region from the state in Figure 24 in the second aspect of the present invention.

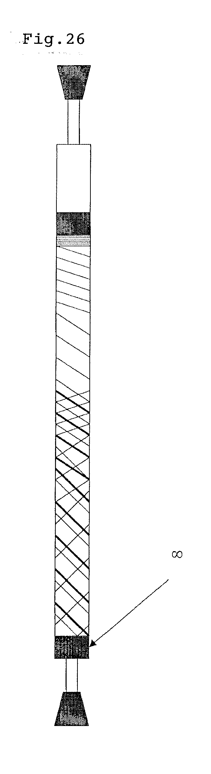

Figure 26 shows the state when the strand terminal region is covered with a shrink tube and fixed by heat shrinkage and then the strand is cut, in the second aspect of the present invention.
Figure 27 shows the state when four kinds of resin tubes different in Shore D hardness for the outer layer are placed densely in the second aspect of the present invention.
Figure 28 shows the state when the shrink tube at the winding initial end of the strand of reinforcement material layer is removed in the second aspect of the present invention.
Figure 29 shows the state when a radiopaque marker is placed in the second aspect of the present invention.
Figure 30 shows the state when the length of the inner layer tube distal region is adjusted in the second aspect of the present invention.
Figure 31 shows the state when the outer layer resin tube is slided to the position of the inner layer tube distal region or a position slightly closer to the proximal end in the second aspect of the present invention.
Figure 32 shows the state when a shrink tube is placed around the outer layer resin tube to the end in the second aspect of the present invention.
Figure 33 shows the state when the distal region of the outer layer resin tube is processed into a rounded shape with the position of the distal region almost identical with the position of the distal region of the inner layer tube in the second aspect of the present invention.
Figure 34 shows the distal end of the tube composite and a mold for heating the distal end in the second aspect of the present invention.
Figure 35 shows the state when the distal end of the tube composite is placed and molded under heat in the distal region-molding mold in the second aspect of the present invention.
Figure 36 shows a catheter tube obtained in the second aspect of the present invention.
Figure 37 shows the state when the outer layer is removed in the distal region of Figure 36 in the second aspect of the present invention.
Figure 38 is a schematic diagram showing the rigidity/flexibility balance of the catheter tube in the second aspect of the present invention.
Figure 39 shows the state when multiple structures having an inner layer tube wound by a reinforcement material layer connected to each other in series by welding of the metal core wire that are wound around a reel in the second aspect of the present invention.
Figure 40 shows the state when the outer layer is formed by switching extrusion in the second aspect of the present invention.
Figure 41 shows the state when the shrink tube at the winding initial end of the strand of reinforcement material layer in the distal region is removed and the length of the distal region of the inner layer tube is adjusted in the second aspect of the present invention.
Figure 42 shows the state when a radiopaque marker is placed in the second aspect of the present invention.
Figure 43 shows the state when a resin tube for distal outer layer is placed and covered with a shrink tube in the second aspect of the present invention.
Figure 44 shows a catheter tube obtained in the second aspect of the present invention.

### EXPLANATION OF REFERENCES

- 1: Metal core wire
- 2: Inner layer tube
- 3: Strand
- 4: Shrink tube
- 5: Winder
- 6: Region close to the initial end
- 7: End of wound strand
- 8: Shrink tube
- 9a: Outer layer tube having the maximum Shore D hardness
- 9b: Outer layer tube having high Shore D hardness
- 9C: Outer layer tube having low Shore D hardness
- 9d: Outer layer tube having the lowest Shore D hardness
- 10: Marker
- 11: Inner layer tube distal region
- 12: Position of sliding outer layer tube
- 13: Shrink tube
- 14: Inner layer tube
- 15: Round molded area
- 16: Reel
- 17: Extrusion mold
- 18: Extruder
- 19: Marker
- 20: Resin tube for outer layer at the most distal end
- 21: Shrink tube
- 22: Outer layer tube at the distal end
- a: Interval of strand
- θ: Tilt angle of strand
- α: Catheter-tube distal region

### BEST MODE OF CARRYING OUT THE INVENTION

Hereinafter, favorable embodiments of the medical catheter tube according to the present invention and the method of producing the same will be described with reference to drawings. These drawings are schematic drawings showing the characteristics of the configuration of the present invention, and the length and diameter of each region is arbitrary, if the catheter tube can be used favorably as a catheter tube for medical treatment.

First, a first aspect of the present invention, a catheter tube in which a strand constituting the reinforcement material layer is wound with turning, will be described.

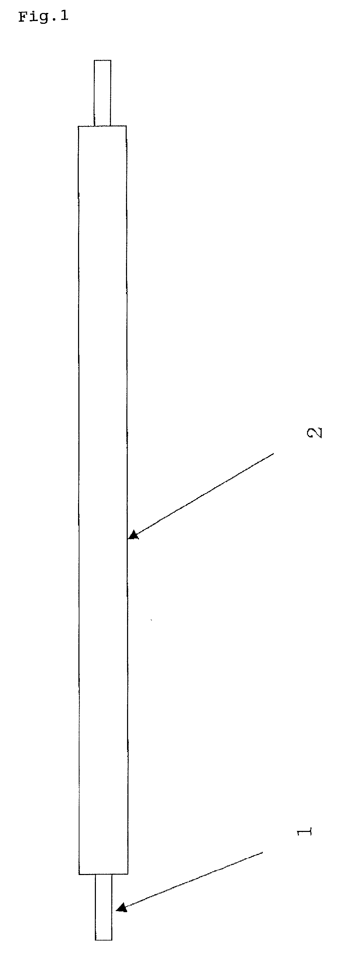

As shown in Figure 1, an inner layer tube 2 covering a metal core wire 1 is prepared. In Figure 1, the catheter tube in the left is the proximal region (hereinafter, referred to as proximal region), and that in the right is the distal region (hereinafter, referred to as distal region).

The inner layer tube is not particularly limited, if it is a resin tube, and examples of the resins for the layer include fluorine resins such as polytetrafluoroethylene, tetrafluoroethylene-perfluoroalkyl vinyl ether copolymers, tetrafluoroethylene-hexafluoropropylene copolymers, and ethylene-tetrafluoroethylene copolymers; polyolefins such as polypropylene, polyethylene, and ethylene-vinyl acetate copolymers; polyamides; polyesters such as polyethylene terephthalate and polybutylene terephthalate; polyurethanes, polyvinyl chlorides, polystyrene resins, polyimide and other resins, and the mixtures thereof. The resin tube is preferably made of polytetrafluoroethylene or a tetrafluoroethylene-perfluoroalkyl vinyl ether copolymer, for making the final product show favorable lubricity, for example, to a guide wire passing through the inner layer tube and favorable positioning efficiency include favorable guide wire compatibility.

The inner layer tube covering the metal core wire preferably has sufficiently high adhesiveness to the metal core wire. For improvement in adhesiveness between the inner and outer layer tubes in the later step of coating an outer layer tube, the surface of the inner layer tube may be roughened or modified additionally by a mechanical method (e.g., by rubbing the inner layer tube surface for example with sand paper in the direction perpendicular to the axial direction) and/or a chemical method (by using chemicals such as sodium naphthalene and dimethylether), and/or an electrical method such as plasma processing.

The distal region of the inner layer tube may be processed mechanically or chemically to make the outer diameter decline gradually in the direction toward the tip.

The reinforcement material layer is formed by winding a strand around the inner layer tube.

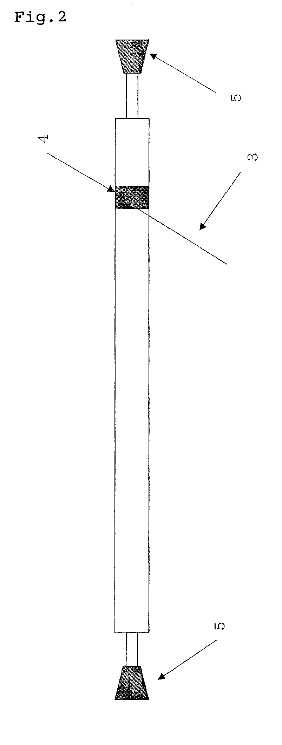

As shown in Figure 2 the winding initial end of a strand 3 is fixed with a shrink tube 4 that contracts in its diameter by heating on the inner layer tube covering the metal core wire shown in Figure 1 , and both ends of the wire is set in a winder 5.

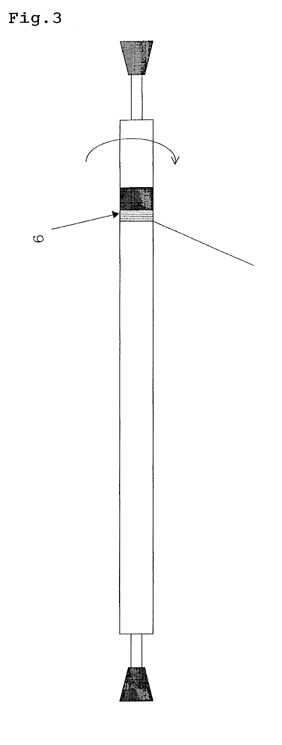

As shown in Figure 3, the strand 3 is wound around the inner layer densely in a very small region 6 from the initial end by the winder. The dense winding means that neighboring wires are wound without spaces between them.

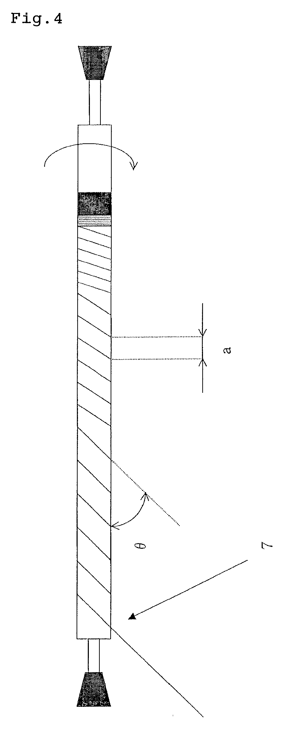

As shown in Figure 4, the strand is the wound at a constant tilt angle and/or a constant interval in the winding region in the direction toward the proximal region from the dense winding region and at a tilt angle and/or interval changing continuously or stepwise in the region further toward the proximal region. The distance between the strand is designated as "a" , while the tilt angle of the strand, θ. As for the interval a of strand and the tilt angle θ of strand, a is preferably smaller and θ larger, as the strand becomes closer to the distal region.

The material for the strand constituting the reinforcement material layer is not particularly limited, if it has a rigidity sufficient for giving reinforcement effect, and examples thereof include various metal materials such as stainless steel, copper, tungsten, nickel, titanium, piano wire, Ni-Ti alloy, Ni-Ti-Co alloy, Ni-Al alloy, Cu-Zn alloy, ultraelastic alloys such as Cu- Zn-X alloy (for example, X: Be, Si , Sn, Al, orGa), and amorphous alloys; polyesters such as polyethylene terephthalate (PET), polybutylene terephthalate (PBT), and polymethylene terephthalate (PPT); polyolefins such as polyethylene and polypropylene; hard polyvinyl chloride, polyamide, polyimide, polystyrene, thermoplastic polyurethane, polycarbonate, ABS resins, acrylic resins, polymethyl methacrylate (PMMA), polyacetal (PA), polyarylate, polyoxymethylene (POM), high-tension polyvinyl alcohol,fluoroplastics,polyvinylidene fluoride (PVdF), polytetrafluoroethylene, ethylene-vinyl acetate hydrolysates (EVOH), polysulfone, polyether sulfone, polyether ketone, polyphenylene oxide, polyphenylene sulfide, aromatic polyaramides such as Kevlar, polymer alloys thereof, carbon fiber, and glass fiber. Among these materials, preferable is stainless steel, because it is less X-ray visible than the radiopaque marker, which is effective for assuring the X-ray visibility of the radiopaque marker installed later, and also superior in processability and cost efficiency, and non-toxic.

The strand may be monofilament or fiber assembly (for example twine) of the material above. The strand preferably has a diameter giving a needed and sufficient reinforcement effect depending on the constituent material, and, for example, when a metal material described above is used, the diameter is preferably approximately 5 to 50 µm. The strand may be used alone or as combination of multiple strands.

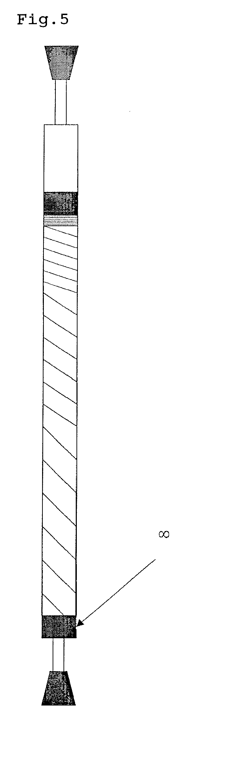

Because the strand rigid enough to give sufficient reinforcement effect is often elastic as well, the winding terminal 7 of the strand is preferably cut off for prevention of unwinding after it is wound with a plastic metal wire such as of aluminum or with a shrink tube 8 that contracts in diameter by heating, and the cut strand, fixed therewith by heating, as shown in figure 5.

The winder 5 that winds the strand to form the reinforcement material layer has functions to wind the strand fed from a strand-supplying unit helically around the outer surface of inner layer tube and to change the tilt angle to the catheter tube axis of strand and/or the interval in the axial direction of catheter tube continuously and/or stepwise by adjusting the relative positions horizontally between the inner layer tube and the strand-supplying unit and the relative traveling speed and/or the relative rotational speed between the inner layer tube and the strand-supplying unit.

Alternatively, the winder 5 for winding the strand may rotate the inner layer tube covering metal core wire while the strand-supplying unit is fixed, and yet alternatively, rotate the strand-supplying unit while the inner layer tube covering metal core wire is fixed. For prevention of dislocation of the strand wound around the inner layer tube during winding, it is preferable to apply a certain tension between the strand and the inner layer tube.

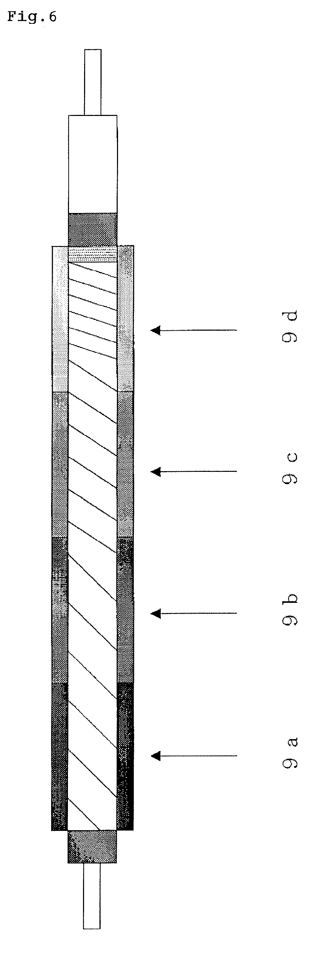

As shown in Figure 6, the structure of the inner layer tube wound with strand is removed from the winder, and outer layer tubes, resin tubes 9a to d, having a Shore D hardness of two or more are attached thereto in that order from the proximal region to the distal region. In Figure 6, four kinds of resin tubes different in Shore D hardness are attached in the order that the Shore D hardness gradually declines in the direction from the proximal region to distal region. Specifically, the Shore D hardness of the outer layer resin tubes in Figure 6 is in the order of 9a > 9b > 9C > 9d. Resin tubes having a Shore D hardness of approximately 20 to 80 are used favorably. When only one kind of outer layer tube having a particular Shore D hardness is used, the outer layer tube having a particular Shore D hardness may be attached densely as it is divided into pieces. Preferably, there is a thin interval between the structure of the inner layer tube wound with strand and the outer layer resin tube for prevention of disturbance of the strand of reinforcing material layer.

Examples of the materials for the outer layer resin tube include various elastomers such as polyamide elastomer, polyester elastomer, polyurethane-elastomer, polystyrene elastomer, fluorine elastomer, silicone rubber, latex rubbers, and the compounds of two or more thereof.

Typical examples of the polyamide elastomers include block copolymers containing an aliphatic or aromatic polyamide (such as nylon 6, nylon 64, nylon 66, nylon 610, nylon 612, nylon 46, nylon 9, nylon 11, nylon 12, or an N-alkoxymethyl-modified nylon), a hexamethylenediamine-isophthalic acid condensation polymer, or a meta-xyloyldiamine-adipic acid condensation polymers as its hard segment, and a polymer such as polyester or polyether as its soft segment, as well as polymer alloys (polymer blend, graft polymerization, random polymerization, etc.) of the polyamide with a more flexible resin, polyamides above softened with a plasticizer or the like, and the mixtures thereof.

Typical examples of the polyester elastomers include block copolymers of a saturated polyester such as polyethylene terephthalate or polybutylene terephthalate and a polyether or polyester, as well as the polymer alloys thereof, saturated polyesters above softened with a plasticizer or the like, and the mixtures thereof.

Favorably used from the viewpoints of processability and flexibility are polyamide elastomers, and typical examples thereof include PEBAX manufactured by Elf Atochem and others.

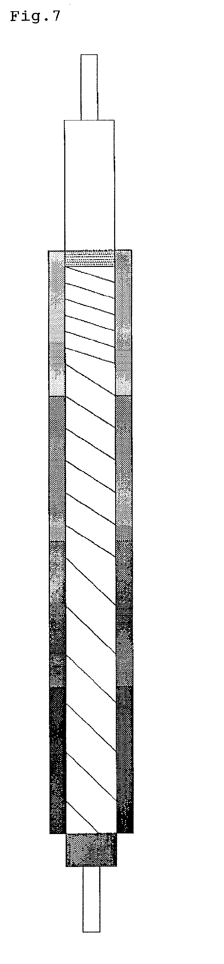

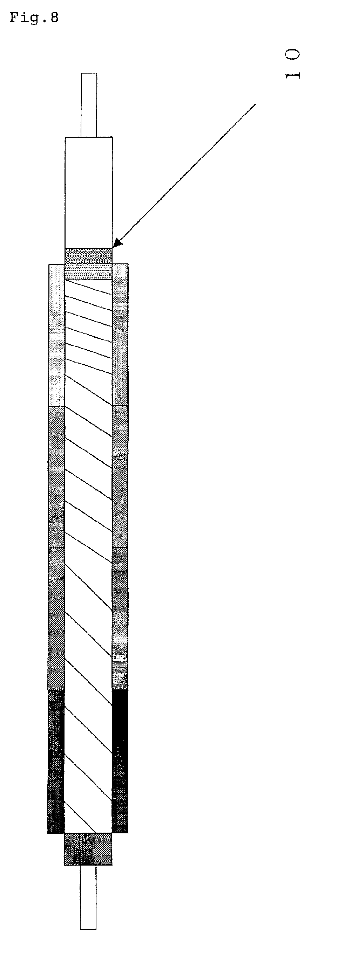

As shown in Figure 7, the shrink tube fixing the strand of reinforcement material layer at the winding initial end is then removed. A radiopaque marker 10 is then attached thereto, as shown in Figure 8. The marker is preferably a dense-wound coil of a material higher in X-ray impermeability and thus X-ray visibility such as platinum (Pt), Pt-Ir alloy, Pt-W alloy, Pt-Ni alloy, gold, or silver. The diameter is preferably approximately 5 to 50 µm.

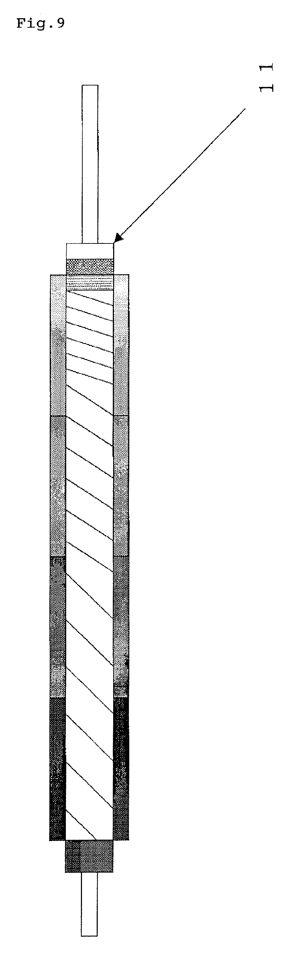

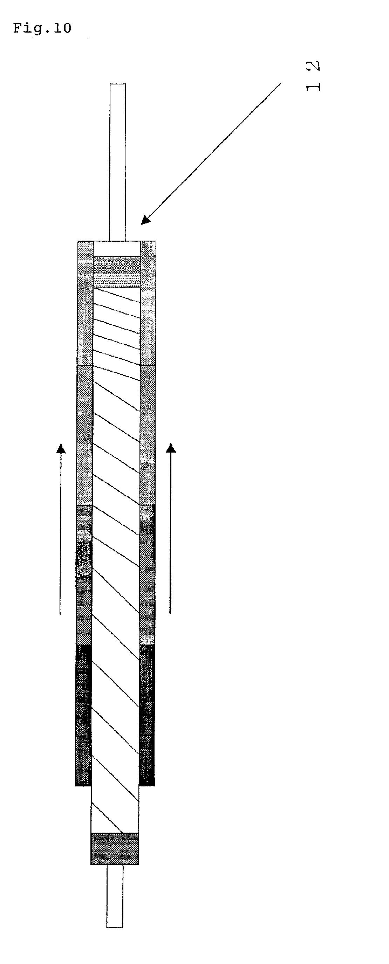

As shown in Figure 9, the length of the inner layer tube distal region 11 is adjusted, and, as shown in Figure 10, the outer layer resin tube is slided to the position of the inner layer tube distal region or a position slightly closer to the proximal end 12.

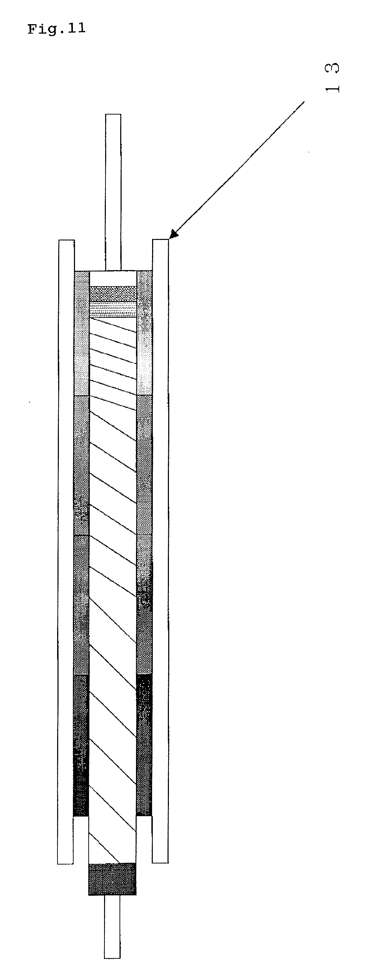

Then as shown in Figure 11 a shrink tube 13 that contracts in diameter by heating is placed over the outer layer resin tube. The material for the shrink tube is preferably polytetrafluoroethylene, a perfluoroethylene-propene copolymer, or the like.

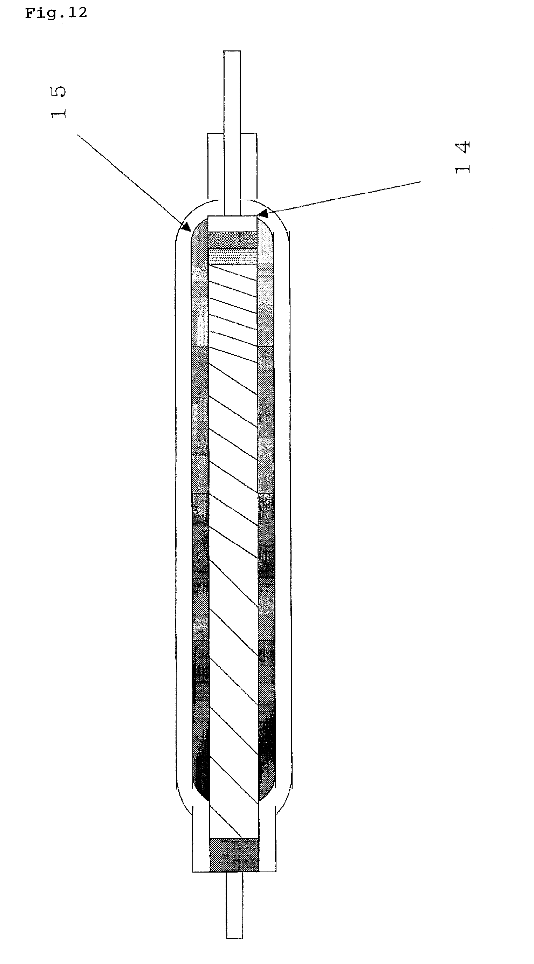

The shrink tube is then heated to a temperature allowing it to shrink by a heater and/or by application of high-frequency electromagnetic wave, allowing integration of the inner layer tube, reinforcement material layer, and outer layer tube. Then as shown in Figure 12, the distal region of the outer layer resin tube is also processed into a rounded shape 15 with the position of the distal region almost identical with the position of the distal region of the inner layer tube 14.

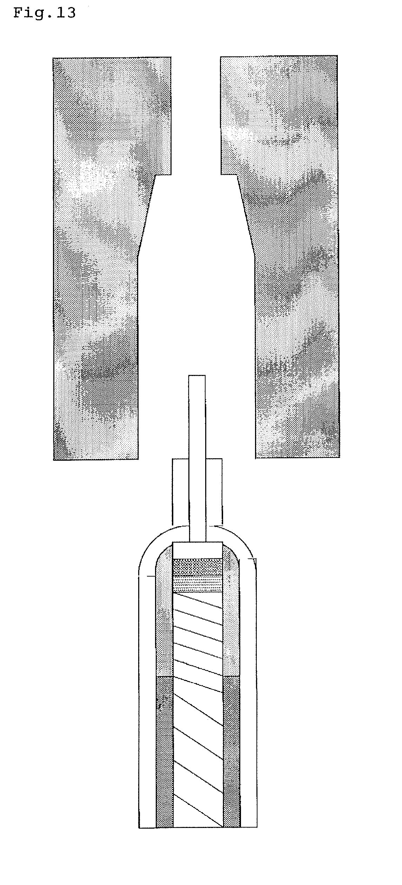

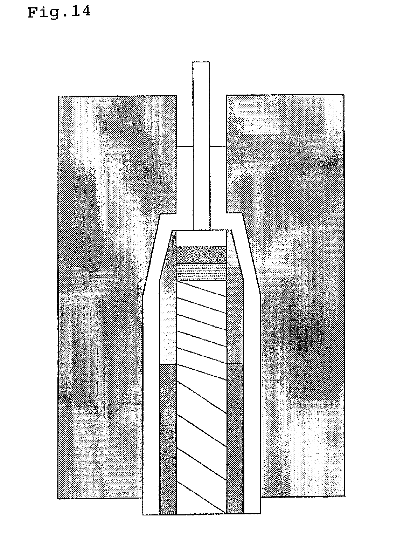

In forming the outer layer resin tube distal region into the tapered shape, the shrink tube is shrunk and the composite is additionally molded under heat in a heated mold of Figure 13, as shown in Figure 14.

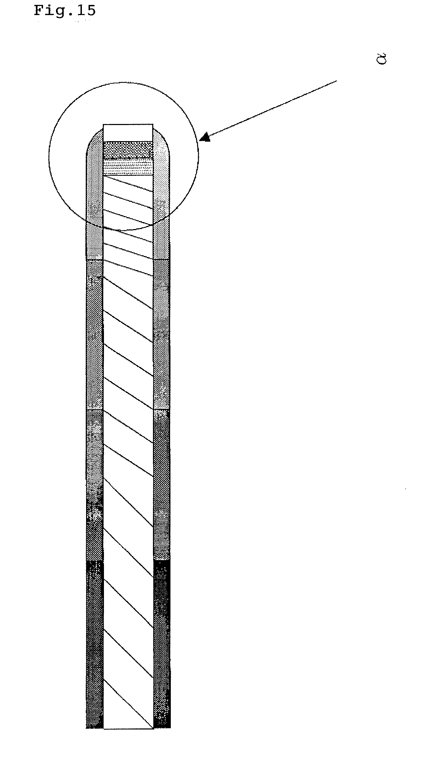

Finally as shown in Figure 15, the shrink tube is removed; the metal core wire is pulled off; and additionally for shaping, the inner layer, reinforcement material layer, and outer layer at the proximal terminal were cut off with a means such as disk-shaped diamond cutter revolving at high speed, and the proximal terminal cross section is finished into a single plane, to give a catheter tube.

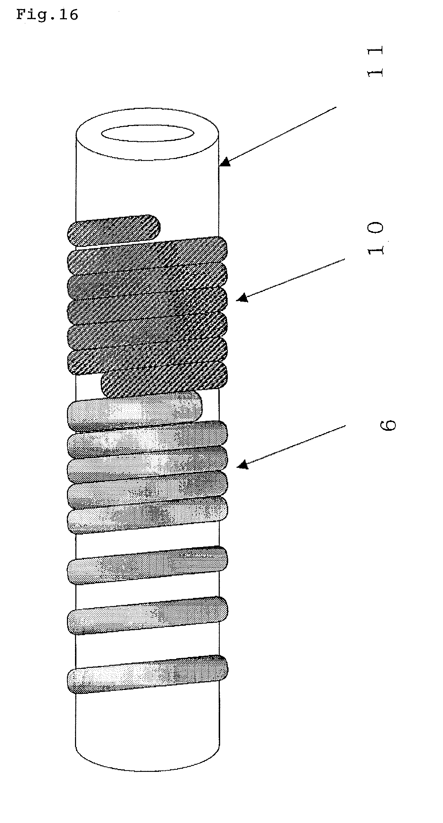

The catheter tube is characterized in that, when seen with the outer layer removed, a densely-wound X-ray non-permeable marker 10 is placed at the position closer to the proximal region from inner layer tube distal region 11 in the α region of Figure 15 and a dense-winding region 6 of the reinforcing strand, very close to the winding initial end and next to the marker above, as shown in Figure 16. In such a structure, the distal region of catheter tube shows favorable X-ray visibility and also soft distal-region property.

Although not shown in the Figure, the surface of the catheter tube is preferably covered with a hydrophilic (or water-soluble) polymer substance. In this way, it is possible to reduce the friction coefficient of the catheter, making it more lubricant, and to increase the lubricity of the catheter tube further even when the outermost surface of the catheter tube becomes in contact with blood, physiological saline, or the like, and consequently, to improve insertion efficiency, compatibility, kink resistance, and stability further. Examples of the hydrophilic polymer substances include the following natural or synthetic polymer substances and the derivatives thereof. Particularly favorable are cellulosic polymer substances (such as hydroxypropylcellulose), polyethylene oxide-based polymer substances (such as polyethylene glycol), maleic anhydride-based polymer substances (maleic anhydride copolymers such as methylvinylether-maleic anhydride copolymer), acrylamide-based polymer substances (such as polyacrylamide), and water-soluble nylons, because these materials give a low friction coefficient consistently.

Although not shown in the Figure, a hub in a suitable feature is connected to the proximal end, to give a catheter tube for medical treatment in the most desirable feature.

The catheter tube for medical treatment may be used as it is, but part of it may be curved if needed, for example, by heating with a heater before use.

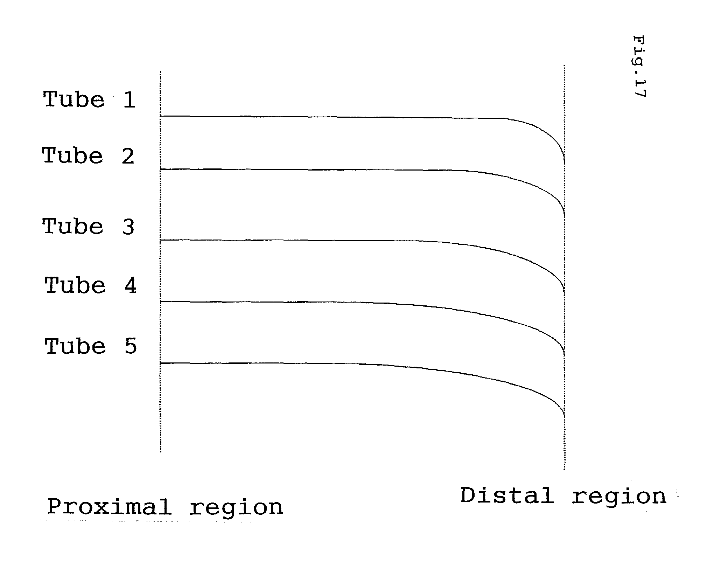

It is possible to give the catheter tube a higher latitude in adjusting the tilt of rigidity and flexibility and the adjusting the rigidity/flexibility balance according to access route, by properly adjusting the length, interval and tilt angle of the reinforcement material and the order and length of the resin tubes different in Shore D hardness. The rigidity/flexibility balance is difference of the position of the high-flexibility region in the distal region or of the position of change in bending strength, as shown in Figure 17. Figure 17 shows that the straight region is higher in rigidity than the distal region and yet has a favorable flexibility as well. Proper adjustment and selection of the rigidity/flexibility balance gives various advantages, and, for example, a catheter tube similar to the catheter tube 1 shown in Figure 17 transmits the state in distal region directly at high sensitivity and transmits torque efficiently, while a catheter tube similar to the tube 5 allows easier insertion into deeper area via a complicated route and transmits the intention of surgeon's operational method to various affected parts more effectively.

The outer layer tube may be formed instead by the following method.

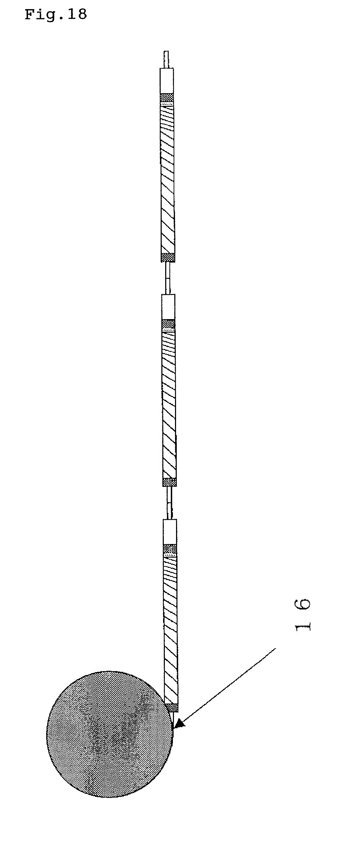

Multiple structures of the reinforcement material layer covering an inner layer tube shown in figure 5 are connected to each other in series by welding of the metal core wires, as shown in Figure 18, and the composite is wound around a reel 16.

Then, the outer layer tube is formed by extrusion molding in such a manner that the Shore D hardness becomes one or more, or the Shore D hardness decreases gradually in the direction from the proximal region to the distal region if the Shore D hardness is varied, and thus, the inner layer tube, reinforcement material layer, and outer layer tube are integrated.

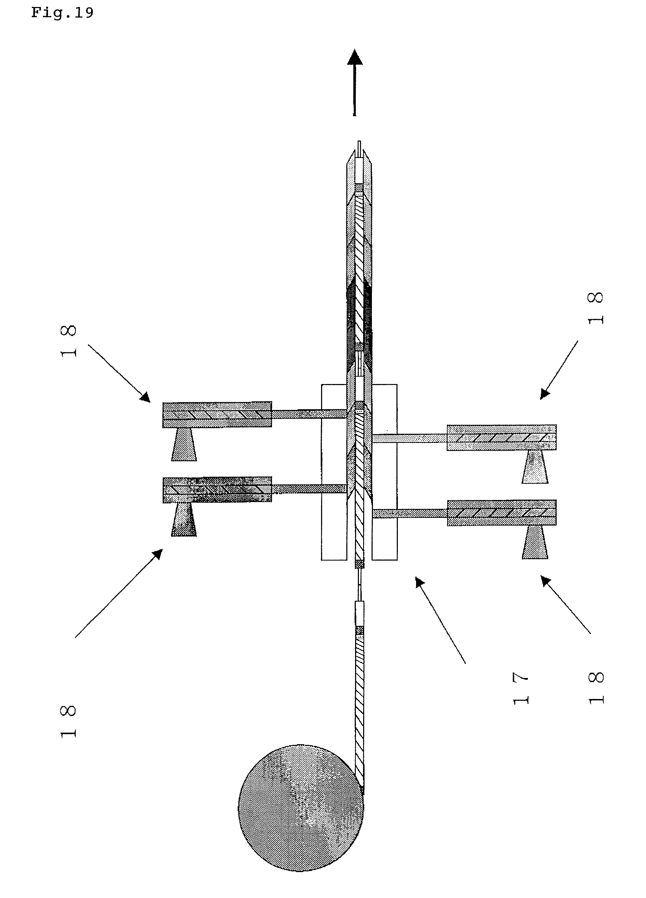

In the case of multi-phase resin coating, for example by using four resins different in Shore D hardness, four extruders 18 are connected to one extrusion die 17, the resins are extruded respectively from the four extruders intermittently, forming an outer layer tube while the condition is adjusted to give a desirable outer diameter, as shown in Figure 19. Although not shown in the Figure, four extruders may be connected to a die having a valve mechanism and resins different in Shore D hardness be extruded continuously one by one, forming the outer layer tube, while supply thereof into the extrusion channel is switched.

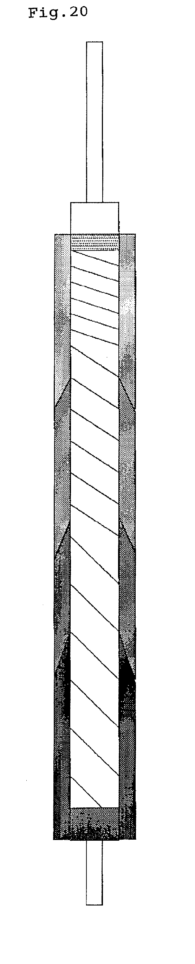

After the outer layer coating extrusion, the metal core wire is cut off one by one; the shrink tube fixing the winding initial end of the strand of the reinforcement material layer in the distal region is removed; and the length of the distal region of inner layer tube is adjusted, as shown in Figure 20.

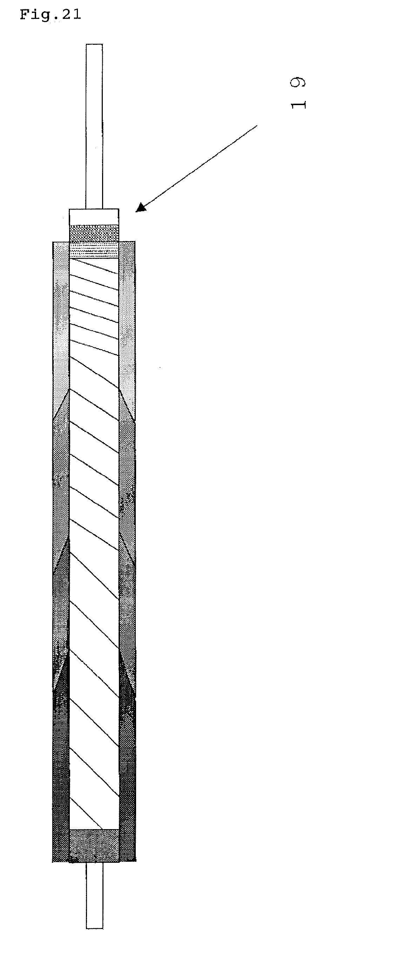

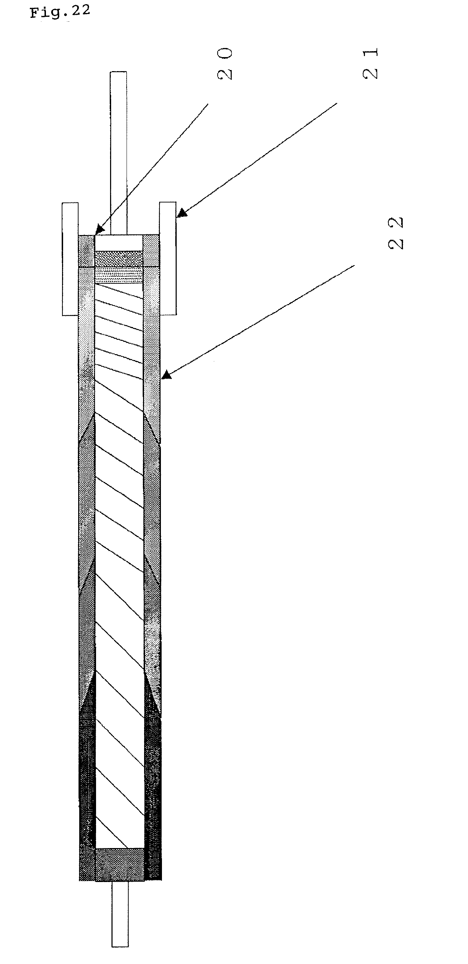

Then as shown in Figure 21, a radiopaque marker 19 is placed thereon. A resin tube 20 for the outer layer at the most distal end is then placed thereon and covered with a shrink tube 21, as shown in Figure 22. Then, the material for the resin tube 20 is preferably the same as that for the outer layer 22 or has a Shore D hardness lower than that of the outer layer 22.

Removing the shrink tube 21 after heat shrinkage, removal of the metal core wire by pulling, cleavage at the proximal end, and subsequent adjustment of the cross section by cutting gives a catheter tube shown in 23. Although not shown in the Figure, the distal region may be processed into a rounded shape with the position of the distal region almost identical with the position of the distal region of the inner layer tube, by a method similar to that for the configuration B above. Similarly to the distal region in Figure 16, the distal region of the catheter tube is characterized in that, when seen with the outer layer removed, a densely-wound X-ray non-permeable marker is placed at a position closer to the proximal region from inner layer tube distal region and a dense-winding region of reinforcing strand very close to the winding initial end and next to the marker above, as shown in Figure 16. In the structure, the distal region of catheter tube shows favorable X-ray visibility and also soft distal-region property.

Hereinafter, a second aspect of the present invention, a catheter tube having a strand for reinforcement material layer that is wound with turning, will be described. Similarly to the first aspect of the invention above, an inner layer tube 2 covering a metal core wire 1 is prepared first as shown in Figure 1, and a strand 3 is wound around the inner layer tube 2 as shown in Figures 2 and 3.

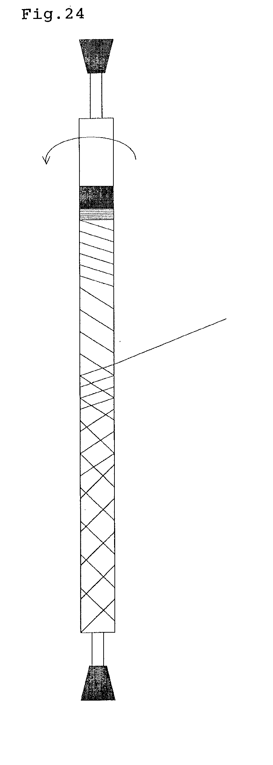

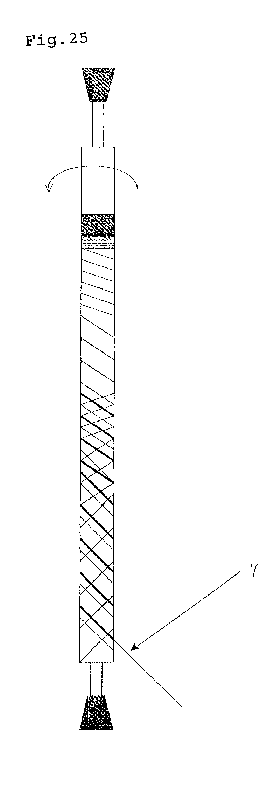

When the strand is wound with turning, as shown in figure 24, the strand is wound in the direction toward the distal end and then backward toward the proximal region, as shown in Figure 25.

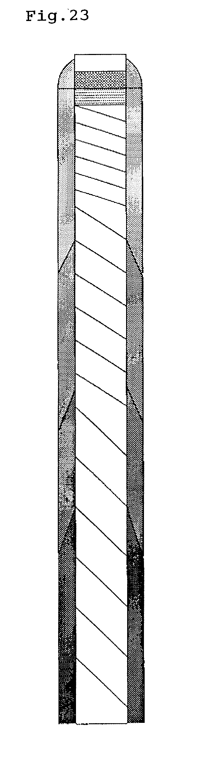

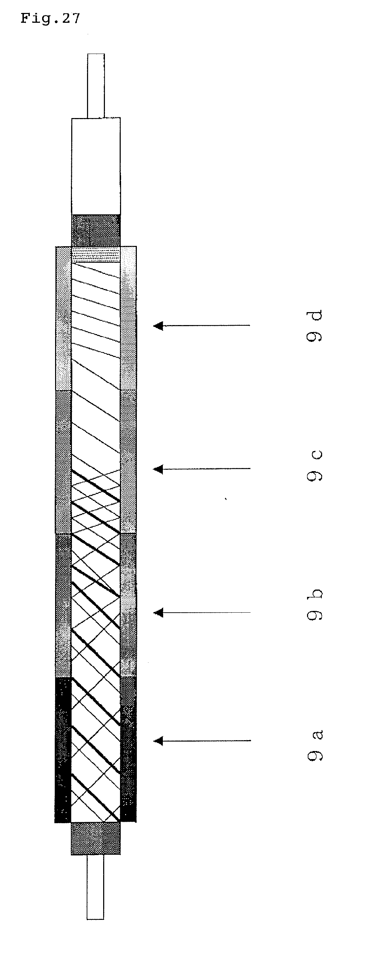

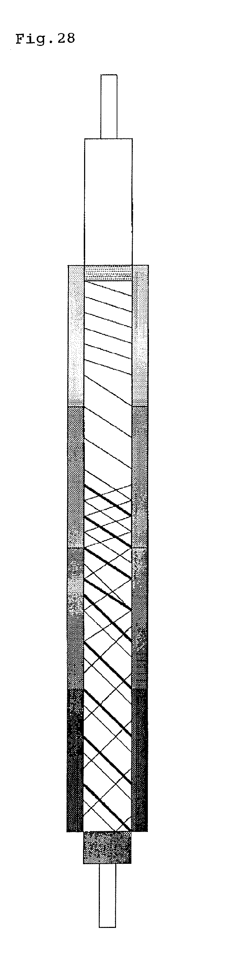

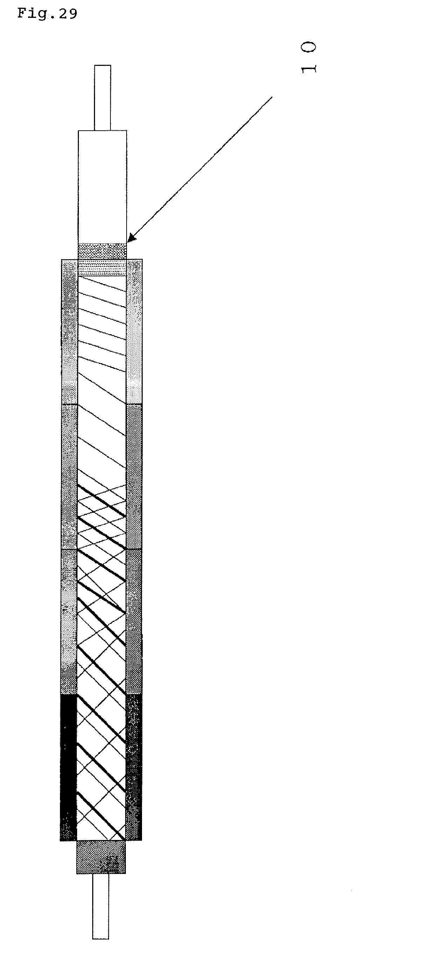

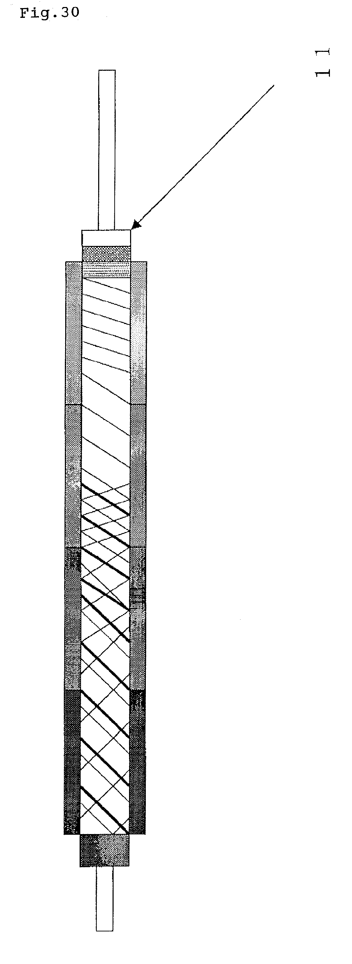

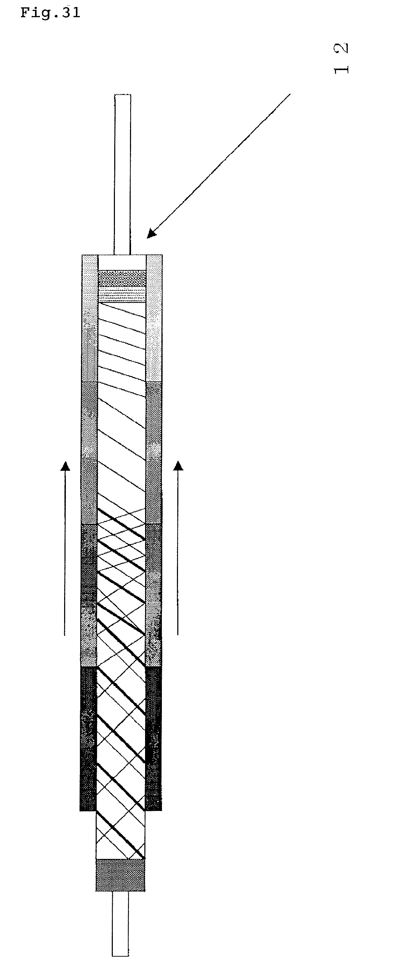

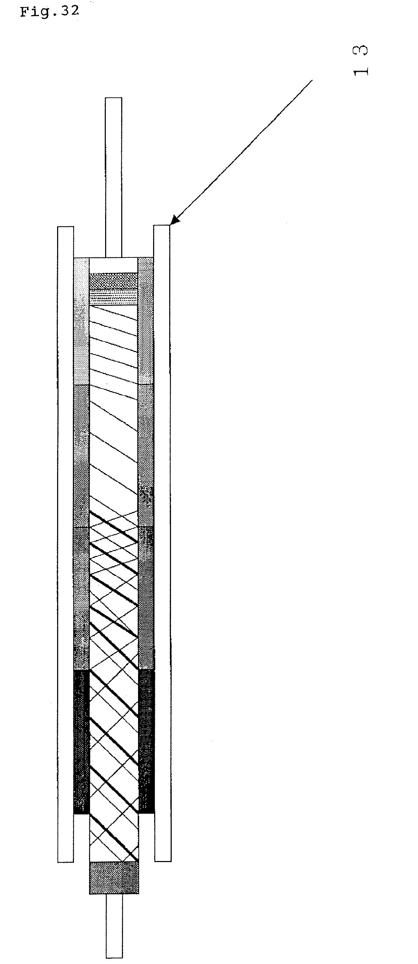

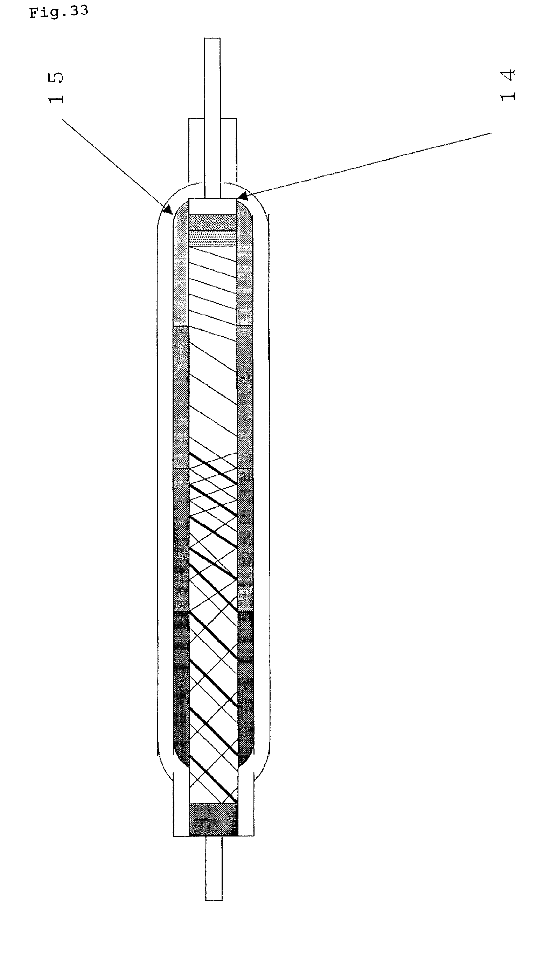

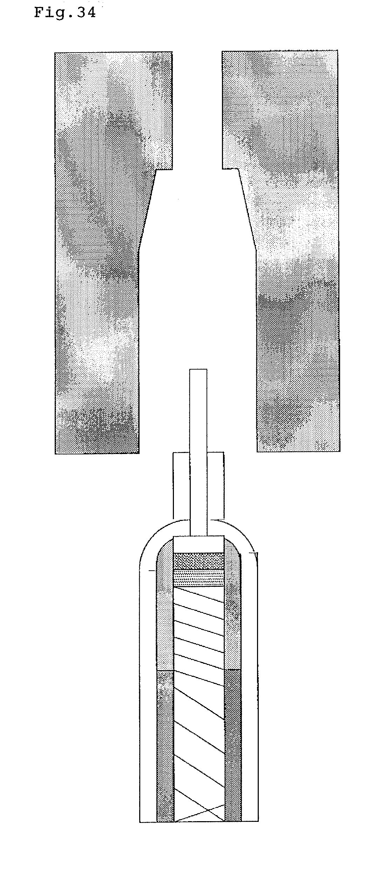

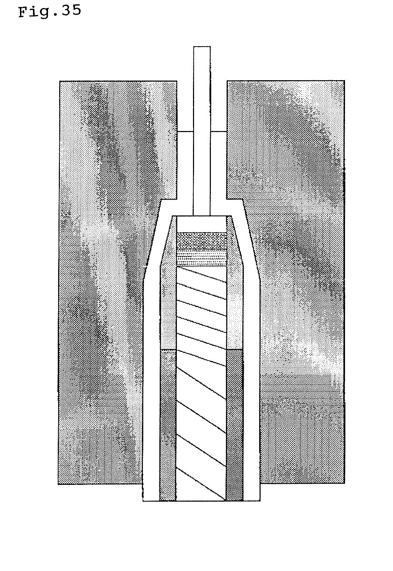

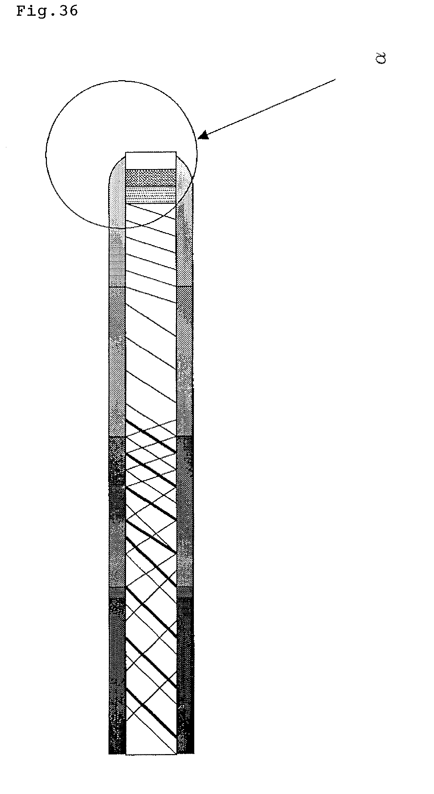

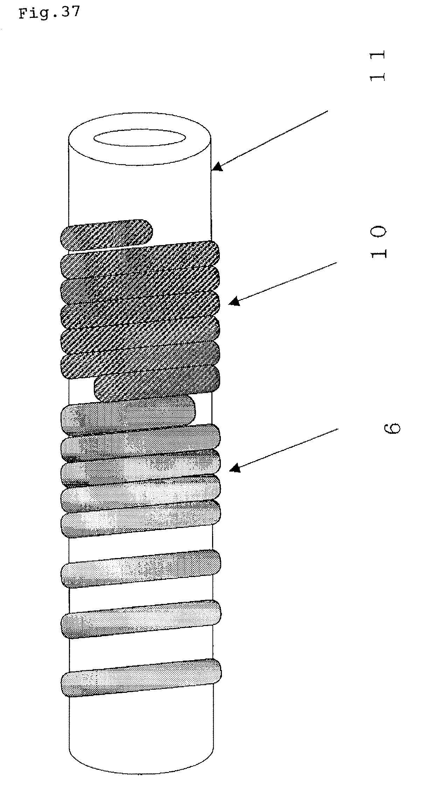

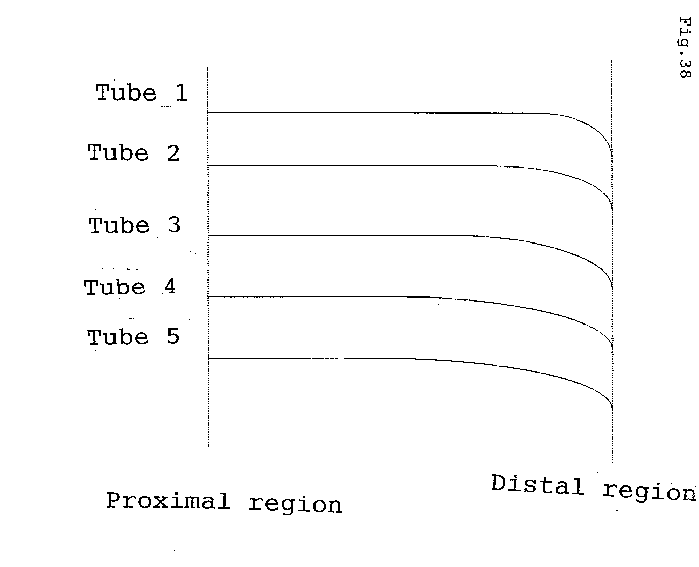

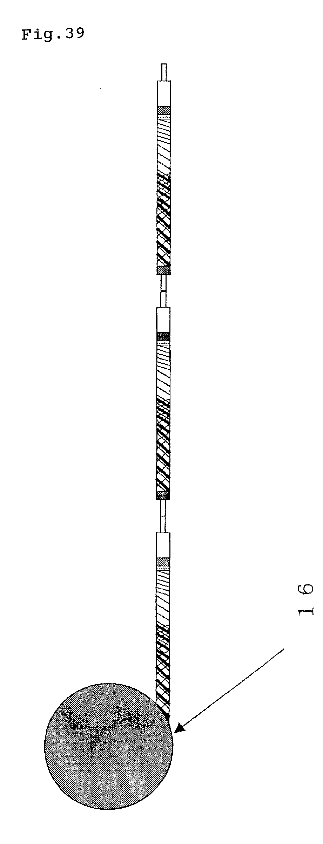

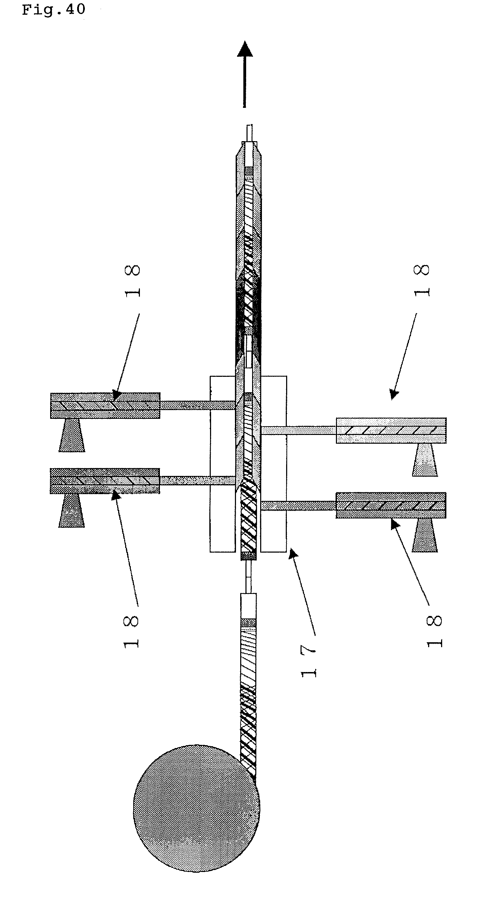

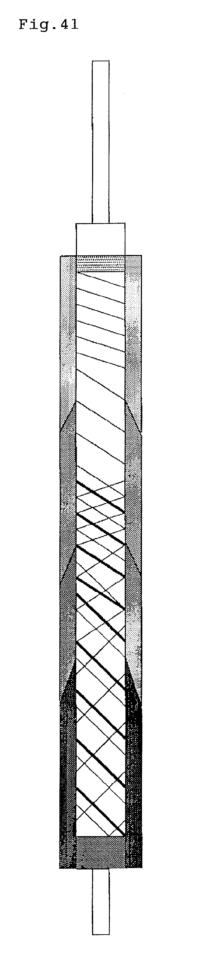

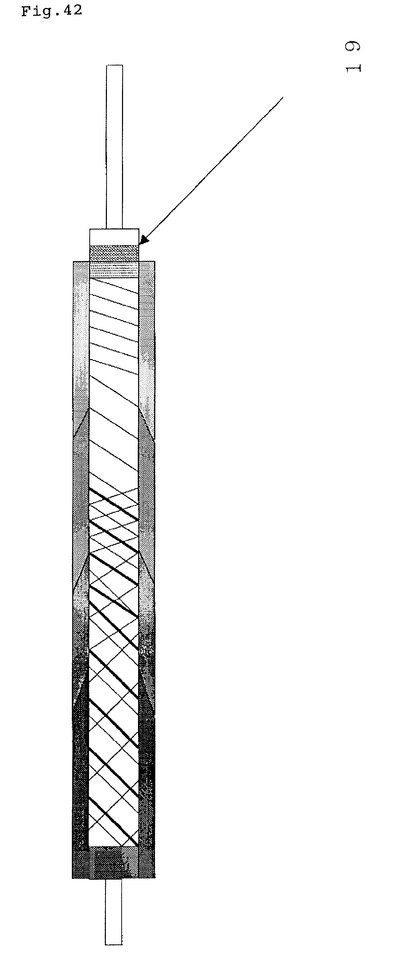

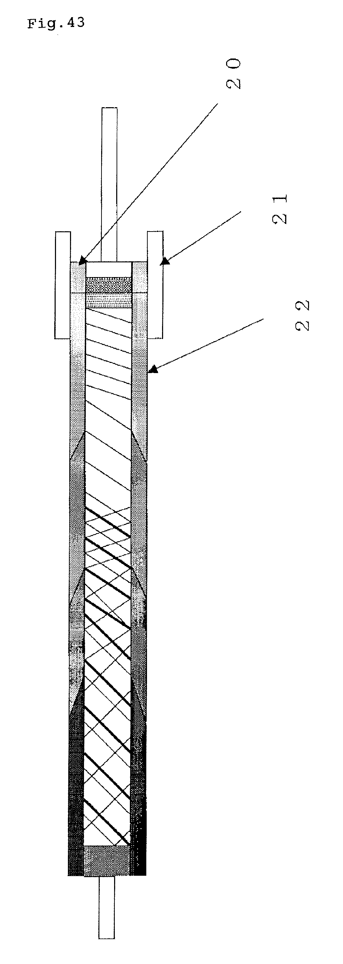

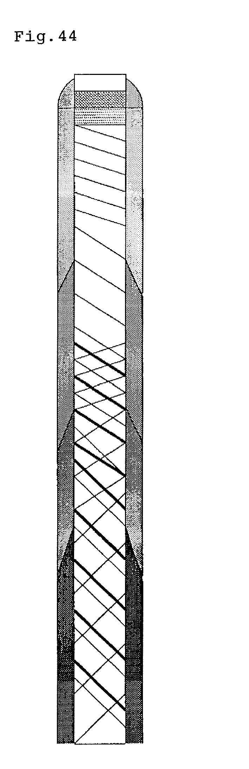

The catheter tube in a favorable embodiment of the second aspect is then prepared in a similar manner to the first aspect. Figures 6 to 23 described in the first aspect of the invention correspond respectively to Figures 27 to 44 in the second aspect of the invention.

## Claims

1. A catheter tube for medical treatment, comprising:
an inner layer resin tube; a reinforcement material layer of a strand for adjustment of flexibility and improvement in kink resistance, torque-transmitting efficiency, and insertion efficiency wound around the inner layer tube; a marker of a radiopaque metal wire wound around the inner layer tube; and an outer layer resin tube covering the reinforcement material layer and the marker, wherein:
the catheter tube includes a distal area and a proximal area;
the distal area includes a tip region;
the proximal area includes a distal region, an intermediate region, and a proximal region;
in the proximal area,
the strand constituting the reinforcement material layer is wound densely in the distal region, and
the interval and tilt angle of winding are changed at least partially and the winding extends to the proximal region of the intermediate region;
in the distal area,
the marker is placed in a proximal region of the distal area, and the strand constituting the marker is wound densely, and
the strand is not wound in the region of the distal area other than the marker region; and
the flexural rigidity declines stepwise or continuously in the direction from the proximal region to the distal region in the proximal area because of presence of the reinforcing material layer.

2. A catheter tube for medical treatment, comprising
an inner layer resin tube, a reinforcement material layer of a strand for adjustment of flexibility and improvement in kink resistance, torque-transmitting efficiency, and insertion efficiency wound around the inner layer tube, a marker of a radiopaque metal wire wound around the inner layer tube, and an outer layer resin tube covering the reinforcement material layer and the marker, wherein:
the catheter tube includes a distal area and a proximal area;
the distal area includes a tip region;
the proximal area includes a distal region, an intermediate region, and a proximal region;
in the proximal area,
the strand constituting the reinforcement material layer is wound densely in the distal region,
and the winding extends to the proximal region while the interval and tilt angle of winding are changed at least partially in the intermediate region, and turns from the proximal region, then turns again from the distal end of the proximal region, and terminates in the proximal region;
in the distal region,
the marker is placed in a proximal region of the distal area, and the strand constituting the marker is wound densely, and
the strand is not wound in the region of the distal area other than the marker region; and
the flexural rigidity declines stepwise or continuously in the direction from the proximal region to the distal region in the proximal area because of presence of the reinforcing material layer.

3. The catheter tube for medical treatment according to Claim 1 or 2, wherein:
the catheter tube in the intermediate region of the proximal area includes a first intermediate area in the distal end side and a second intermediate area in the proximal region side;
the strand constituting the reinforcement material layer is wound at a constant tilt angle and/or a constant interval in the first intermediate area; and
the strand is wound at an tilt angle and/or a interval changing continuously or stepwise in the second intermediate area.

4. The catheter tube for medical treatment according to Claim 1 or 2, wherein the Shore D hardness of the resin tubes constituting the outer layer tube declines continuously or stepwise in the direction from the proximal region to the distal region

5. The catheter tube for medical treatment according to Claim 4 , wherein the rigidity/flexibility balance of the catheter tube is adjusted arbitrarily by winding of the wire of reinforcement material layer and stepwise decrease in the Shore D hardness of the resin tubes constituting the outer layer tube from the proximal region to the distal region.

6. The catheter tube for medical treatment according to Claim 1 or 2, wherein the inner- and outer-layer tubes are connected to each other via the reinforcement material layer and the marker.

7. The catheter tube for medical treatment according to Claim 1 or 2, wherein the inner layer tube is made of a resin lubricant to the guide wire or the like penetrating therein.

8. The catheter tube for medical treatment according to Claim 1 or 2, wherein the outer layer tube is molded into a rounded or tapered shape while the outer diameter thereof is changed and/or the inner and outer diameters of the inner layer tube changed in the tip region of the distal area.

9. The catheter tube for medical treatment according to Claim 1 or 2, wherein the outer layer tube is made hydrophilic by coating.

10. A method of producing the catheter tube for medical treatment according to Claim 1 or 2, comprising:
preparing an inner layer tube;
forming a marker on the inner layer tube,
winding a strand fed from a strand-supplying unit around the outer surface of the inner layer tube and forming a reinforcement material layer by changing the relative traveling speed and/or the relative traveling direction of the inner layer tube and the strand-supplying unit, and/or changing the relative traveling direction of the inner layer tube and the strand-supplying unit, while changing the tilt angle and interval of the strand to the catheter tube axis continuously and/or stepwise along the axial direction of the catheter tube; and
covering the inner layer tube, the marker, and the reinforcement material layer with an outer layer tube.

11. The method of producing a catheter tube according to Claim 10, wherein:
two or more resin tubes are used for the outer layer tube;
the resin tubes respectively have a Shore D hardness of 2 or more; and
the resin tubes are placed around the structure having a reinforcement material layer and a marker formed on the outer surface of inner layer tube, forming the outer layer tube in such a manner that the Shore D hardness of the resin tubes of the catheter tube declines stepwise in the direction from the proximal region to the distal region.

12. The method of producing a catheter tube according to Claim 11, further comprising:
preparing an outer layer tube by placing the resin tubes;
covering and heating all of the resin tubes with a shrink tube; and
integrating the inner layer tube, the reinforcing material layer, the marker and the outer layer tube;
then, molding the tip region of the outer layer tube into a rounded or tapered shape; and
obtaining a catheter tube for medical treatment by removing the shrink tube.

13. The method of producing a catheter tube according to Claim 10, wherein
the outer layer tube is formed by coating one or more resins on the structure having a reinforcement material layer and a marker on the outer surface of the inner layer tube by extrusion in such a manner that the Shore D hardness becomes 2 or more and declines thereon in the direction from the proximal region to the distal region.
